Europäisches Patentamt

European Patent Office

Office européen des brevets

(11) Publication number: **0 150 969**
**B1**

# EUROPEAN PATENT SPECIFICATION

(45) Date of publication of patent specification: **20.07.88**

(51) Int. Cl.⁴: **C 07 C 2/00**

(21) Application number: **85300398.6**

(22) Date of filing: **22.01.85**

(54) Process for converting light olefins into heavier hydrocarbons.

(30) Priority: **01.02.84 US 576179**

(43) Date of publication of application:
**07.08.85 Bulletin 85/32**

(45) Publication of the grant of the patent:
**20.07.88 Bulletin 88/29**

(84) Designated Contracting States:
**BE DE FR GB IT NL SE**

(56) References cited:
EP-A-0 127 284
DE-A-2 400 946
US-A-4 433 185
US-A-4 450 311
US-A-4 456 779

(73) Proprietor: **MOBIL OIL CORPORATION**
150 East 42nd Street
New York New York 10017 (US)

(72) Inventor: **Chung, Hweng Hsia**
11 Somerset Place
Matawan New Jersey 07747 (US)
Inventor: **Owen, Hartly**
5-Rivervieuw Terrace
Belle Mead New Jersey 08502 (US)
Inventor: **Wright, Bernard, Stanley**
13 Shagbark Lane
East Windsor New Jersey 08520 (US)

(74) Representative: **Cooper, John Anthony et al**
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This invention relates to a process for converting light olefins into higher hydrocarbons such as gasoline-range and/or distillate-range fuels, and more particularly to a technique for operating a catalytic reactor system with ethene-rich feedstock and an effluent fractionation-recovery system.

The continued development of catalytic hydrocarbon conversion processes has created interest in utilizing olefinic feedstocks, such as petroleum refinery streams rich in lower olefins, for producing $C_5^+$ gasoline, diesel fuel and other distillate fuels. In addition to the basic work derived from ZSM-5 type zeolite catalyst research, a number of discoveries have contributed to the development of an industrially applicable process known as the "Mobil Olefins to Gasoline/Distillate" (MOGD). Process which is of significance as a safe, environmentally acceptable technique for utilizing refinery streams that contain lower olefins, especially $C_2$—$C_5$ alkenes, and may supplant conventional alkylation units. In U.S. Patents 3,960,978 and 4,021,502, there is described the conversion of $C_2$—$C_5$ olefins, alone or together with paraffinic components, into higher hydrocarbons over crystalline zeolites having controlled acidity. Improved MOGD processing techniques are also described in U.S. Patents 4,150,062, 4,211,640 and 4,227,992.

The conversion of lower olefins, especially propene and butenes, over H-ZSM-5 is effective at moderately elevated temperatures and pressures. The conversion products are sought as liquid fuels, especially the $C_5^+$ aliphatic and aromatic hydrocarbons. Olefinic gasoline is produced in good yield by the MOGD process and may be recovered or recycled to the reactor system for further conversion to distillate-range products.

As a consequence of the relatively low reactivity of ethene with known zeolite oligomerization catalysts known distillate-mode reactor systems designed completely to convert a large ethylenic component of feedstock are required to be of a much larger size than comparable reactor systems for converting other lower olefins. However, under severe conditions of temperature and pressure, 75% or more of ethene can be converted in a single pass. Recycle of a major amount of ethene gas from the reactor effluent can result in significant increases in equipment size, especially recycle compressors.

Olefinic feedstocks may be obtained from various sources, including fossil fuel processing streams, such as gas separation units, cracking of $C_2^+$ hydrocarbons, coal byproducts, alcohol or ether conversion, and various synthetic fuel processing streams. The cracking of ethane and conversion of effluent is described in U.S. Patent 4,100,218 and the conversion of ethane into aromatics over Ga-ZSM-5 is described in U.S. Patent 4,350,835. Olefinic effluent from the fluidized catalytic cracking of gas oil or the like is also a valuable source of olefins suitable for exothermic conversion by the MOGD process.

The present invention is based on the observation that an olefin-oligomerization process utilizing $C_2^+$ olefinic feedstock can be operated efficiently to fractionate the effluent for ethene recovery.

According to the invention, there is provided a continuous process for converting an ethene-rich olefinic feedstock into heavier liquid hydrocarbons comprising the steps of (a) combining the feedstock stream with a liquid hydrocarbon stream containing a major amount of gasoline range hydrocarbons including $C_5^+$ olefins; (b) contacting the combined feedstock-gasoline stream from step (a) at an elevated temperature and pressure in a reaction zone with an oligomerization catalyst comprising a shape-selective, medium pore zeolite to convert at least a portion of the olefinic components into heavier hydrocarbons; (c) cooling oligomerization reaction effluent from step (b) to condense at least a portion of the heavier hydrocarbons; (d) separating the cooled and partially condensed effluent stream from step (c) into an ethene-rich vapor stream and a condensed liquid hydrocarbon stream; (a) fractionating the condensed hydrocarbon stream from step (d) to provide a gasoline stream, a distillate product stream and a light hydrocarbon vapor stream containing unreacted ethene; (f) contacting the ethene-rich vapor stream from the separation step (d) and the light hydrocarbon vapor stream from step (e) under sorption pressure conditions with a cooled liquid portion of the gasoline hydrocarbon from step (e) to sorb ethene into the liquid gasoline stream; and pressurizing and recycling the sorbed ethene and gasoline stream to step (a) for combining with an ethene-rich feedstock.

An olefinic feedstock, such as $C_2$—$C_4$ olefins derived from alcohol dehydration or catalytic cracker (FCC) effluent, may be employed as a suitable feedstock rich in ethene, propene and butenes for the process. Typically, the olefinic stock consists essentially of $C_2$—$C_6$ aliphatic hydrocarbons containing a major fraction of monoalkenes in the essential absence of dienes or other deleterious materials. The process may employ various volatile lower olefins as feedstock, with oligomerization of alpha-olefins being preferred for either gasoline or distillate production. Preferably the olefinic feedstream contains at least 50 to 75 mole % $C_2$—$C_4$ alkenes.

Process conditions, catalysts and equipment suitable for use in the MOGD process are described in U.S. Patents 3,960,978, 4,021,502 and 4,150,062. Hydrotreating and recycle of olefinic gasoline are described in U.S. Patent 4,211,640 and other pertinent process features are described in U.S. Patents 4,227,992 and 4,450,311.

The catalyst used in the process comprises a medium pore, shape selective crystalline silicaceous metal oxide material such as an acid ZSM-5 type zeolite. These materials are commonly referred to as aluminosilicates or porotectosilicates; however, the acid function may be provided by other tetrahedrally coordinated metal oxide moieties, especially Ga, B, Fe or Cr. Commercially available aluminosilicates such

2

as ZSM-5 are nevertheless preferred.

These catalytic materials are effective in oligomerizing lower olefins, especially ethene, propene and butene-1, into higher hydrocarbons. The unique characteristics of acid ZSM-5 are particularly suitable for use in the MOGD system. Effective zeolites include those described in U.S. Patents 4,430,516 and 4,465,884. A preferred catalyst is an extrudate (1—5 mm) comprising 65 weight % HZSM-5 and 35% alumina binder, having an acid cracking activity (alpha) of 160 to 200.

The medium pore crystalline zeolites used in such catalysts are characterized by a pore dimension greater than 0.5 mm i.e., capable of sorbing paraffins having a single methyl branch as well as normal paraffins, and a structural silica to alumina mole ratio of at least 12.

However, it is preferred to use zeolites having silica to alumina mole ratios of at least 30 up to 30,000 or more.

The especially preferred zeolites are ZSM-5, ZSM-5/ZSM-11 intermediate, ZSM-11, ZSM-12, ZSM-23, ZSM-35, ZSM-38 and ZSM-48. ZSM-5 is described in U.S. Patents 3,702,886, Re. 29,948 and 4,061,724; ZSM-5/ZSM-11 intermediate is described in U.S. Patent 4,229,424; ZSM-11 is described in U.S. Patent 3,709,979; ZSM-12 is described in U.S. Patent 3,832,449; ZSM-23 is described in U.S. Patent 4,076,842; ZSM-35 is described in U.S. Patent 4,016,245; ZSM-38 is described in U.S. Patent 4,046,859; and ZSM-48 is described in European Patent 0015132.

The zeolites may be in the hydrogen form or they may be base exchanged or impregnated to contain a rare earth cation complement. Such rare earth cations comprise Sm, Nd, Pr, Ce and La. It is desirable to calcine the zeolite after base exchange.

The catalyst composition will comprise the catalytically active zeolite together with a binder and is suitably in the form of particles such as pellets or extrudates, and the particle size of the particles may be from 1 to 10 mm.

Binder materials include both synthetic and natural substances, including clays such as bentonite and kaolin, silica and/or metal oxides. The latter may be either naturally occurring or in the form of gelatinous precipitates, sols or gels including mixtures of silica and metal oxides.

A particularly preferred catalyst composition comprises extruded pellets having a diameter of 1 to 3 mm, made by mixing steamed zeolite crystals for example, of silica:alumina=70:1—500:1, with alpha-alumina monohydrate in a proportion of about 2:1 and extruding and calcining the mixture to obtain a dried extrudate having a void fraction of about 30—40%, preferably about 36%.

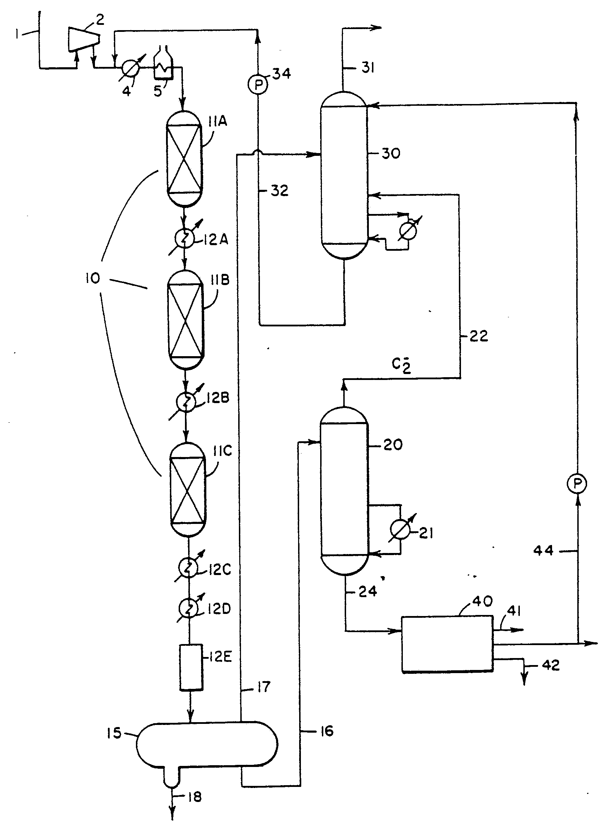

The accompanying drawing is a flow diagram of the process of the invention.

Referring to the drawing, olefinic feedstock is supplied through conduit 1 under steady stream conditions. This $C_2^+$ feedstream is pressurized by compressor 2 and then sequentially heated by passing through process heat exchange unit 4 and furnace 5 to achieve the temperature for catalytic conversion in reactor system 10, including plural reactor vessels 11A, B and C.

The reactor system consists of three downflow fixed-bed series reactors on line with heat exchanger cooling means 12A, B, C, D, E and between and downstream of the reactors. The cooled reactor effluent is separated in a phase separator 15 to provide a condensed $C_5^+$ hydrocarbon liquid stream in conduit 16 and an ethene-rich vapor stream in conduit 17 comprising $C_2$—$C_4$ aliphatic hydrocarbons, along with any other unreacted gaseous components which might be present in the feedstock, such as hydrogen, carbon oxides, methane, nitrogen or other inert gases. Extraneous water may be removed from the system through separator line 18.

Condensed hydrocarbon reactor effluent 16 separated from the effluent vapor is further fractionated. A stripping unit 20 which may be heated by exchanging a reactor effluent stream in reboiler 21, removes a significant fraction of dissolved light gases, including a minor amount of unreacted ethene. The $C_2^-$ stripped gases are passed through conduit 22 to a downstream sorption unit 30. Ethane and heavier hydrocarbons are removed from the recycle loop through stripper 20. This tower may be designed to lose as little ethene as possible while maintaining a reasonable tower bottom temperature. High pressure favors the split between ethene and ethane. Preferably the liquid stripper effluent 24 is debutanized in a fractionation system 40 to provide a $C_4^-$ overhead stream, which is deethanized to provide LPG ($C_3$—$C_4$ alkane) product 41 and light offgas. The $C_5^+$ debutanizer bottom stream is split in an atmospheric distillation tower to provide raw distillate product stream 42 and an olefinic gasoline stream 44 for recycle and/or recovery of a minor amount as raw gasoline product. Details of a suitable fractionation system and other process conditions are described in U.S. Patent 4,456,779.

To recycle unconverted ethene, recycle gasoline is used to selectively absorb it in the ethene absorber 30. Ethene is recovered from the vapor stream 17 leaving the reactor effluent separator and from the stripper overhead 22. The $H_2$, CO, $CO_2$ and $CH_4$ inerts which may enter with the feed are removed in the tower overhead via conduit 31 to prevent their build up in the system.

The gasoline sorbent is an aliphatic hydrocarbon mixture boiling in the normal gasoline range of 50 to 165°C with minor amounts of $C_4$—$C_5$ alkanes and alkenes. Preferably, the total gasoline sorbent stream to ethene sorbate mole ratio is greater than 4:1. The process may be operated with a mole ratio of 0.2 moles to 10 moles of gasoline per mole of $C_2^+$ olefins in the feedstock.

The tower pressure and bottom temperature may be selected such that enough $CO_2$ leaves the system without carrying too much ethylene with it. Ethene absorption efficiency can be improved if $CO_2$ is removed by an optional amine scrubber (not shown) before entering the tower.

3

There is no need for a recycle compressor because all the recovered ethene is dissolved in the recycle gasoline as a sorbate stream 32 and passed by pump 34 to the reactor. Advantageously, the liquid recycle stream is brought to process pressure before being heated to vaporize at least a portion of the olefinic components.

The fractionation towers may employ a plate column in the primary tower and a packed column in the secondary tower, however, the fractionation equipment may also employ vapor-liquid contact means of various designs in each stage including packed beds of Raschig rings, saddles or other solids or low pressure drop valve trays (Glitsch grids). The number of theoretical stages will be determined by the feedstream composition, liquid:vapor (L/V) ratios, desired recovery and product purity.

A typical distillate mode multi-zone reactor system employs inter-zone cooling, whereby the reaction exotherm can be carefully controlled to prevent excessive temperature above the normal moderate range of 260 to 370°C.

Advantageously, the maximum temperature differential across any one reactor is about 30°C and the space velocity (LHSV based on olefin feed) is 0.5 to 1. Heat exchangers provide inter-reactor cooling and reduce the effluent to fractionation temperature. It is an important aspect of energy conservation in the MOGD system to utilize at least a portion of the reactor exotherm heat value by exchanging hot reactor effluent from one or more reactors with a fractionator stream to vaporize a liquid hydrocarbon distillation tower stream, such as the debutanizer reboiler. Optional heat exchangers may recover heat from the effluent stream prior to fractionation. Gasoline from the recycle conduit is pressurized and combined with feedstock, preferably at a mole ratio of 2—3 moles per mole of olefin in the feedstock. It is preferred to operate in the distillate mode at elevated pressure of 4200 to 7000 kPa with a minimum olefin partial pressure of 1200 kPa at the reactor system inlet.

The reactor system contains multiple downflow adiabatic catalytic zones in each reactor vessel. The liquid hourly space velocity (based on total fresh feedstock) is about 1 LHSV. In the distillate mode the molar recycle ratio for gasoline is at least equimolar, based on total olefins in the fresh feedstock and recycle.

The preferred molar ratio of olefinic gasoline to fresh feedstock olefin is at least 2:1. This also assures adequate sorbent for the sorption unit.

Typical reactor conditions are as follows:

TABLE I

Reactor system conversion feedstock and yield

| Feedstock | | Yield on olefin converted | |
|---|---|---|---|
| Component | Wt % | Component | Wt % |
| Inerts | 5.00 | $CH_4$ | 0.10 |
| $CH_4$ | 2.00 | $C_2H_6$ | 3.90 |
| $C_2H_4$ | 81.20 | $C_3H_8$ | 4.00 |
| $C_2H_6$ | 0.62 | $iC_4H_{10}$ | 2.00 |
| $C_3H_6$ | 3.71 | $nC_4H_{10}$ | 2.00 |
| $C_3H_8$ | 0.20 | $iC_5H_{12}$ | 1.32 |
| $iC_4H_{10}$ | 0.25 | $nC_5H_{12}$ | 0.09 |
| $nC_4H_{10}$ | 0.45 | $C_5H_{10}$ | 2.99 |
| $C_4H_8$ | 0.12 | $C_6$-150°C gasoline | 39.60 |
| $iC_5H_{12}$ | 2.31 | 150°C+ distillate | 44.00 |
| $nC_5H_{12}$ | 0.10 | | |
| $C_5H_{10}$ | 1.73 | | 100.00 |
| $C_6^+$ | 2.31 | | |
| | 100.00 | | |

Conversion on feed to reactor

| Olefins | Wt. % |
|---|---|
| $C_2$ | 75 |
| $C_3$ | 95 |
| $C_4$ | 85 |

4

TABLE II
Reactor conditions

| | |
|---|---|
| Space Velocity, LHSV (Based on olefins fed to reactor) | 0.5 |
| Reactor A inlet pressure, kPa | 6300 |
| Minimum Olefin pp at reactor inlet, kPa | 1240 |
| Exothermic Heat of Reaction, kJ/kg olefins converted | 2420 |
| Rate of Heat Release | Uniformly over bed |
| Maximum Allowable T in Reactor, °C | 28 |
| Reactor Inlet Temperature, °C SOC/EOC | 260/370 |
| Gasoline Recycle, Mol/Mol Olefin Feed | 2:1 |
| Coke on Catalyst, wt.% SOC | 0 |
| EOC | 30 |
| Cycle Length, Days | 30 |
| Catalyst | HZSM-5 1.6 mm Extrudate |

More than 90% of ethene is recovered in the above example from the effluent.

## Claims

1. A continuous process for converting an ethene-rich olefinic feedstock into heavier liquid hydrocarbons comprising the steps of

(a) combining the feedstock stream with a liquid hydrocarbon stream containing a major amount of gasoline range hydrocarbons including $C_5^+$ olefins;

(b) contacting the combined feedstock-gasoline stream from step (a) at an elevated temperature and pressure in a reaction zone with an oligomerization catalyst comprising a shape-selective, medium pore zeolite to convert at least a portion of the olefinic components into heavier hydrocarbons;

(c) cooling oligomerization reaction effluent from step (b) to condense at least a portion of the heavier hydrocarbons;

(d) separating the cooled and partially condensed effluent stream from step (c) into an ethene-rich vapor stream and a condensed liquid hydrocarbon stream;

(e) fractionating the condensed hydrocarbon stream from step (d) to provide a gasoline stream, a distillate product stream and a light hydrocarbon vapor stream containing unreacted ethene;

(f) contacting the ethene-rich vapor stream from the separation step (d) and the light hydrocarbon vapor stream from step (e) under sorption pressure conditions with a cooled liquid portion of the gasoline hydrocarbon from step (e) to sorb ethene into the liquid gasoline stream; and pressurizing and recycling the sorbed ethene and gasoline stream to step (a) for combining with an ethene-rich feedstock.

2. A process according to Claim 1, wherein gasoline is recycled at a molar ratio of gasoline to fresh feedstock olefin of at least 2:1.

3. A process according to Claim 1 or Claim 2, wherein the reaction zone contains a catalyst comprising acid ZSM-5 type zeolite.

4. A process according to any one of Claims 1 to 3, wherein the combined feedstock/gasoline stream is heated to a temperature of 260 to 370°C and contacted with the oligomerization catalyst at a pressure of 5000 to 7000 kPa with a minimum olefin partial pressure at the reactor inlet of 1200 kPa.

5. A process according to any one of Claims 1 to 4, wherein the feedstock space velocity is 0.5 to 1.0 LHSV, based on total olefins contacted with the oligomerization catalyst.

## Patentansprüche

1. Kontinuierliches Verfahren zur Umwandlung eines ethenreichen olefinischen Ausgangsmaterials in schwerere flüssige Kohlenwasserstoffe, welches die Schritte umfaßt:

a) Kombination des Ausgangsmaterialstromes mit einem flüssigen Kohlenwasserstoffstrom, der eine Hauptmenge an Kohlenwasserstoffen im Benzinbereich enthält, die $C_5^+$-Olefine einschließen,

b) Kontakt dieses kombinierten Stromes von Ausgangsmaterial und Benzin vom Schritt a) bei erhöhter

5

Temperatur und erhöhtem Druck in einer Reaktionszone mit einem Oligomerisierungskatalysator, der einen formselektiven Zeolith mit mittleren Poren umfaßt, um zumindest einen Teil der olefinischen Komponenten in schwerere Kohlenwasserstoffe umzuwandeln,

c) Abkühlen des Abflusses der Oligomerisierungsreaktion vom Schritt b), um zumindest einen Teil der schwereren Kohlenwasserstoffe zu kondensieren,

d) Trennung des abgekühlten und teilweise kondensierten Abflußstromes vom Schritte c) in einen ethenreichen Dampfstrom und einen kondensierten flüssigen Kohlenwasserstoffstrom,

e) Fraktionieren des kondensierten Kohlenwasserstoffstroms vom Schritt d), um einen Benzinstrom, einen Destillatproduktstrom und einen Dampfstrom leichter Kohlenwasserstoffe, der unreagiertes Ethen enthält, zu liefern,

f) Kontakt des ethenreichen Dampfstromes vom Trennungsschritt d) und des Dampfstromes leichter Kohlenwasserstoffe vom Schritt e) bei Sorptionsdruckbedingungen mit einem abgekühlten flüssigen Anteil des Benzinkohlenwasserstoffs vom Schritt e), um Ethen im flüssigen Benzinstrom zu sorbieren, und Komprimieren und Rezirkulieren des Stromes von Benzin und sorbiertem Ethen zum Schritt a) zur Kombination mit dem ethenreichen Ausgangsmaterial.

2. Verfahren nach Anspruch 1, worin das Benzin bei einem Molverhältnis von Benzin zum frischen Olefinausgangsmaterial von mindesten 2:1 rezirkuliert wird.

3. Verfahren nach Anspruch 1 oder 2, worin die Reaktionszone einen Katalysator enthält, der einen sauren Zeolith vom ZSM-5-Typ umfaßt.

4. Verfahren nach einem der Ansprüche 1 bis 3, worin der kombinierte Strom von Ausgangsmaterial/Benzin auf eine Temperatur von 260 bis 370°C erwärmt wird und mit dem Oligomerisierungskatalysator bei einem Druck von 5000 bis 7000 kPa bei einem minimalen Partialdruck des Olefins am Reaktoreinlaß von 1200 kPa in Kontakt gebracht wird.

5. Verfahren nach einem der Ansprüche 1 bis 4, worin die Raumgeschwindigkeit des Ausgangsmaterials, bezogen auf die gesamten Olefine, die mit dem Oligomerisierungskatalysator in Kontakt gebracht werden, 0,5 bis 1,0 LHSV beträgt.

**Revendications**

1. Un procédé continu de conversion d'une charge oléfinique riche en éthène en hydrocarbures liquides plus lourds comprenant les étapes suivantes:

(a) on réunit le courant de charge à un courant d'hydrocarbures liquides contenant une quantité majeure d'hydrocarbures bouillant dans l'intervalle des essences comprenant des oléfines en $C_5^+$;

(b) on met le courant charge-essence réuni provenant de l'étape (a), à une température et à une pression élevées, dans une zone de réaction, au contact d'un catalyseur d'oligomérisation contenant une zéolite sélective de forme à pores moyens, pour convertir au moins une partie des composants oléfiniques en hydrocarbures plus lourds;

(c) on refroidit l'effluent de la réaction d'oligomérisation provenant de l'étape (b) pour condenser au moins une partie des hydrocarbures plus lourds;

(d) on sépare le courant d'effluent refroidi et partiellement condensé provenant de l'étape (c) en un courant de vapeur riche en éthène et un courant d'hydrocarbures liquides condensés;

(e) on fractionne le courant d'hydrocarbures condensés provenant de l'étape (d) pour obtenir un courant d'essences, un courant de distillats et un courant de vapeur d'hydrocarbures légers contenant de l'éthène qui n'a pas réagi;

(f) on met le courant de vapeur riche en éthène provenant de l'étape de séparation (d) et le courant de vapeur d'hydrocarbures légers provenant de l'étape (e), dans des conditions de pression de sorption, au contact d'une portion liquide refroidie des essences provenant de l'étape (e) pour sorber l'éthène dans le courant des essences liquides; et on met sous pression et on recycle l'éthène sorbé et le courant d'essences vers l'étape (a) pour les réunir à la charge riche en éthène.

2. Un procédé selon la revendication 1, dans lequel on recycle l'essence dans un rapport molaire essence/charge neuve d'oléfines d'au moins 2/1.

3. Un procédé selon la revendication 1 ou la revendication 2, dans lequel la zone de réaction contient un catalyseur comprenant une zéolite de type ZSM-5 acide.

4. Un procédé selon l'une quelconque des revendications 1 à 3, dans lequel le courant essence/charge réuni est chauffé à une température de 260 à 370°C et mis au contact du catalyseur d'oligomérisation à une pression de 5 000 à 7 000 kPa, la pression partielle minimum de l'oléfine à l'entrée du réacteur étant de 1 200 kPa.

5. Un procédé selon l'une quelconque des revendications 1 à 4, dans lequel la vitesse spatiale de la charge est égale à 0,5 à 1,0 VSHL, sur la base du total des oléfines mises au contact du catalyseur d'oligomérisation.

0 150 969